(19) Europäisches Patentamt
European Patent Office
Office européen des brevets

(11) Publication number: **0 092 278**
**A1**

(12) **EUROPEAN PATENT APPLICATION**

(21) Application number: **83200528.4**

(22) Date of filing: **13.04.83**

(51) Int. Cl.³: **A 01 N 43/50**
// C07D233/94, C07D233/93, C07D233/92, C07D233/58

(30) Priority: **14.04.82 EP 82103136**

(43) Date of publication of application: **26.10.83**
**Bulletin 83/43**

(84) Designated Contracting States: **AT BE CH DE FR GB IT LI LU NL SE**

(71) Applicant: **Gist - Brocades N.V., Wateringseweg 1 P.O. Box 1, NL-2600 MA Delft (NL)**

(72) Inventor: **Akkerboom, Piet Johannes, Dr. J.W. Palthelaan 48, NL-2712 RT Zoetermeer (NL)**
Inventor: **Van Der Stelt, Cornelis, Laurens Reaellaan 24III, NL-2024 BG Haarlem (NL)**
Inventor: **Wegman, Bernardus B.M., Norholm 58, NL-2133 HL Hoofddorp (NL)**
Inventor: **Tan, Hong Sheng, Buizerdstraat 94, NL-2665 TG Bleiswijk (NL)**

(74) Representative: **Schmieman, Johannes Hendrik et al, Gist-Brocades N.V. Patents & Trademarks Department Wateringseweg 1 P.O. Box 1, NL-2600 MA Delft (NL)**

(54) **Process of combatting fungal infections.**

(57) Ethenylimidazoles of the formula

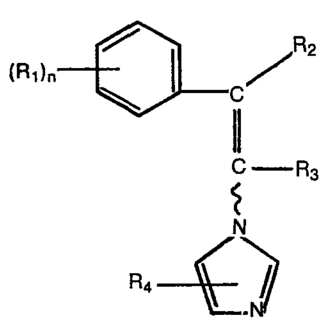

I

in which n is 0, 1 or 2, $R_1$ represents a halogen atom, a trifluoromethyl group or an alkyl or alkoxy group with at most 6 carbon atoms, the two substituents being the same or different when n is 2, $R_2$ represents an alkyl group with 1 to 6 carbon atoms, an alkenyl group with 2 to 4 carbon atoms or a cycloalkyl group with 3 to 6 carbon atoms, $R_3$ represents a hydrogen atom, an alkyl group with 1 to 6 carbon atoms or an alkenyl group with 2 to 4 carbon atoms and $R_4$ represents a hydrogen atom, an alkyl group with 1 to 6 carbon atoms or a nitro group, or an acid addition salt thereof, are used in combatting fungal infections in agricultural products and foodstuffs.



ACTORUM AG

GIST-BROCADES N.V.



Process of combatting fungal infections.

This invention relates to a process of combatting fungal infections in agricultural products and foodstuffs, to compositions to be used in this process and their preparation and to products treated by said process.

Agricultural products, such as fruits, vegetables, seeds and bulbs and foodstuffs like cheese and sausage can be affected by fungi. Some examples of plant pathogen fungi are Penicillum digitatum and P. italicum on oranges, Rhizoctonia solani and Fusarium species on flower bulbs, tubers and grains, Drechslera sorokiniana and D. teres in barley, Ascochyta pisi and Mycosphaerella pinodes in peas, etc.

In the European patent application 81201040.3, filed on September 16, 1981, and included herein by reference, fungicidal compounds are described with the general formula

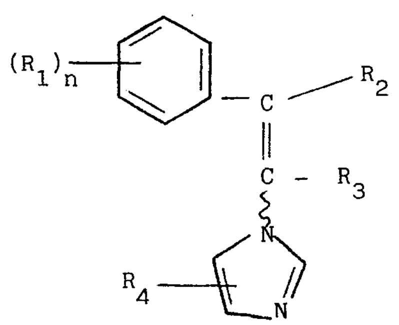

I

in which n is 0, 1 or 2, $R_1$ represents a halogen atom, a trifluoromethyl group or an alkyl or alkoxy group with at most 6 carbon atoms, the two substituents being the same or different when n is 2, $R_2$ represents an alkyl group with 1 to

6 carbon atoms, an alkenyl group with 2 to 4 carbon atoms or a cycloalkyl group with 3 to 6 carbon atoms, $R_3$ represents a hydrogen atom, an alkyl group with 1 to 6 carbon atoms or an alkenyl group with 2 to 4 carbon atoms and $R_4$ represents a hydrogen atom, an alkyl group with 1 to 6 carbon atoms or a nitro group, and their acid addition salts.

It is stated that the compounds are suited for combatting fungal infections in humans and animals. It has now been found that the compounds of formula I can also effectively be used in combatting fungal infections in agricultural products and foodstuffs.

The invention therefore provides a process for combatting fungal infections in agricultural products and foodstuffs which comprises treating them with a compound of formula I or an acid addition salt thereof. The invention also includes the products thus treated. The treatment is preferably carried out with a compound of formula I in which $(R_1)_n$ is 4-chloro or 2,4-dichloro, $R_2$ is ethyl, propyl or sec-butyl and $R_3$ and $R_4$ are hydrogen atoms. Particularly preferred is a treatment with 1-[2-(2,4-dichlorophenyl)-1-pentenyl]-1H-imidazole or an acid addition salt thereof.

The activities of the compounds of formula I have been shown in in vitro experiments with pathogenic moulds and in tests with infected products. In experiments with agricultural products no phytotoxic effects were found.

The invention also comprises formulations for combatting fungal infections in agricultural products and foodstuffs, which comprise as the active ingredient at least one compound of formula I or an acid addition salt thereof.

It is preferred to use liquid formulations, such as solutions, suspensions and emulsions of the active substance in water or an organic solvent.

The free bases of formula I can be dissolved in organic solvents, for instance in hydrocarbon mixtures such as petrol, aromatic compounds like benzene, hydrocarbon chlorides such as chlorobenzenes, chloromethanes and chloroethanes, alcohols like butanol and glycol or strongly polar solvents such as dimethylformamide and dimethylsulfoxide.

It is preferred to use the compounds in the form of their acid addition salts, which can be dissolved in water to which a surfactant (e.g. a glyceride) may be added. Examples of other types of formulations are sprays, in which the active substance is mixed with an aerosol propelling gas such as dichlorodifluoromethane or trichlorofluoromethane, and powders consisting of a mixture of the active substance with a solid carrier, such as kaoline, talc or diatom earth.

The treatment of the products may be carried out, for example, by mixing them with or immersing them in a liquid containing a compound of formula I or an acid addition salt thereof, or to sprinkle or brush them with such a liquid.

It will be understood that the amount of active compound to be used will depend on the kind of product to be treated, particularly on its surface volume. Examples of preferred amounts in proportion to the treated products are

- for seeds (diameters between 0.5 and 10 mm): 1-10,000 ppm by weight, more preferably 10-2000 ppm;
- for tubers and bulbs (diameters between 0.5 and 6 cm): 0.2-1500 ppm by weight, more preferably 2-300 ppm;
- for cheese 0.5-500 mcg/$cm^2$, more preferably 2-20 mcg/$cm^2$.

The following examples 1 through 7 illustrate that the claimed compounds are not phytotoxic. Germination tests were done with seeds of corn, pea, barley, wheat, sugarbeet, garden cress and mangel.

The active ingredients were used as a 10% (w/v) solution in water containing a surface active agent (10g/l of diacetyltartaric acid ester of mono- and diglycerides).

Solution A contained a mixture of Z- and E-isomers of 1-[2-(2,4-dichlorophenyl)-1-butenyl]-1H-imidazole hydrochloride

Solution B contained a mixture of Z- and E-isomers of 1-[2-(2,4-dichlorophenyl)-1-pentenyl]-1H-imidazole hydrochloride

Solution C contained a mixture of Z- and E-isomers of 1-[2-(2,4-dichlorophenyl)-3-methyl-1-pentenyl]-1H-imidazole hydrochloride

Solution D contained the Z-isomer of 1-[2-(4-chlorophenyl)-3-methyl-1-pentenyl]-1H-imidazole hydrochloride;

As references the following commercial fungicide compositions were used:

- Captolate AC, a powder formulation containing 50% of captan and 35% of anthraquinone

- AAtiram AT-S, a powder formulation containing 50% of thiram and 35% of anthraquinone

- Panoctine Plus, a liquid formulation containing 30% (w/v) of guazatine and 2.5% (w/v) of imazalil

- Neo-Voronit, a liquid formulation containing 33.6% (w/v) of sodiumdimethyldithiocarbamate and 0.56% (w/v) of fuberidazole

- AAtiram-75-S, a slurry containing 75% of thiram.

These are recommended fungicide compositions for the disinfection of parent seeds.

Example 1

400 g of corn seeds (Dorina) were put into a flask of ca 750 ml. The size of the flask should be such that only about 66% of the volume is filled with seeds. The test composition was then added with gentle shaking. The flask was tightly closed with a screw cap and then clamped to the edge of a rotating wheel (diameter circa 70 cm). In order to mix thoroughly the wheel was rotated during 20 minutes at a speed of 25-30 rpm.

The germination of the seeds was done in an aluminium tray of about 14x17x3 cm, filled with riversand. The trays, each containing 50 seeds, were incubated in a climatic box at a relative humidity of 95-100%. Eight trays were taken for each treatment. During the incubation the temperature was varied every 24 hours, alternately 30°C during 8 hrs with exposure to artificial daylight and 20°C in darkness during 16 hours. After 7 days of incubation the degree of germination was evaluated.

The results are summarized in Table 1.

Example 2

400 g of peas (Finale) were mixed with test composition and germinated as described in Example 1, except that

the temperature was kept constantly at 20°C during germination and that the degree of germination was evaluated after nine days of incubation.

The results are summarized in Table 2.

Example 3

40 g of barley seeds (Banteng) in a bottle of 200 ml were treated with test composition. The seeds were mixed thoroughly with the fungicide by clamping the closed bottle to a rotating wheel (diameter circa 70 cm) and mixing for 20 minutes.

The seeds were germinated in aluminium trays of 14x17x3 cm filled with silver sand. The trays, each containing 100 seeds were first incubated for three days in darkness at 10°C and then alternately in the dark (16 hours) and in artificial daylight (8 hours) at 20°C with a relative humidity of 95-100%. Four trays were taken for each treatment. After 7 days at 20°C the degree of germination was evaluated.

The results are summarized in Table 3.

Example 4

The procedures described in example 3 were repeated, except that wheat (Caribo) was taken instead of barley.

The results are summarized in Table 4.

Example 5

50 g of sugar beet seeds were put into a bottle of 200 ml. The test composition was added with gentle shaking. The bottle was tightly closed with the screw cap and then clamped to the edge of a turning wheel (diameter circa 70 cm). In order to mix thoroughly the wheel was kept turning during 20 minutes at a speed of 25-30 rpm.

The presoaked seeds (4 hrs, 25°C) were germinated within folded filter paper sheets.

Two seeds were put between each fold, and the filter paper containing 100 seeds was then placed in a plastic tray of 21x15x3 cm. After addition of 130 ml of water the tray was covered with a plastic lid. The trays (each containing 100

seeds) were incubated at 20°C. Four trays were taken for each treatment. After 7 and 14 days of incubation the degree of germination was estimated.

The results are summarized in Table 5.

Example 6

40 g of garden cress seeds (Gromono) were put into a bottle of 100 ml. The test composition was added with gentle shaking. The bottle was closed tightly with the screw cap and then clamped to a turning wheel (diameter circa 70 cm). In order to mix thoroughly the wheel was kept turning during 20 minutes at a speed of 25-30 rpm.

The seeds were germinated on circular filter discs (Type T-300, Schut en Zn, Heelsum, The Netherlands). The filter discs, each with 100 seeds, were preincubated for 2 days at 10°C and then put on a Jacobsen apparatus (or Kopenhagen tables) and incubated at 20°C. Four discs were taken for each treatment. During the preincubation and the incubation the paper discs were covered with a transparant plastic beaker. After 2 and 10 days of incubation the degree of germination was estimated.

The results are summarized in Table 6. The Table shows that there are no phytotoxic effects on garden cress seeds, which generally are highly susceptible to fungicide treatment.

Example 7

The amount of the added ethenylimidazole compound was varied. The following compositions were used:

Solution B: containing 1-[2-(2,4-dichlorophenyl)-1-pentenyl]-1H-imidazole hydrochloride (100 g/l) and diacetyl tartaric acid ester of mono- and diglycerides (10 g/l) in water.

Solution B1: the same as solution B except that it contained 50 g/l instead of 100 g/l of the ethenyl imidazole compound.

Solution B2: the same as solution B, except that it contained 25 g/l instead of 100 g/l of the ethenyl imidazole.

Solution B3: the same as solution B, only with 5

g/l instead of 100 g/l of the ethenyl imidazole.

Solution B4: the same as solution B, only with 0.5 g/l instead of 100 g/l of the ethenyl imidazole.

AAtiram 75-S: a slurry containing 75% of thiram.

50 g of mangel seeds were put into a bottle of 200 ml. With gentle shaking 0.8 ml (16 ml/kg) of test composition was added. The bottle was closed with the screw cap and the seeds were mixed thoroughly with the test composition by clamping the bottle on a turning wheel (20 minutes, 25-30 rpm).

The reference seeds were first treated with 0.8 ml of water, thoroughly mixed, and then mixed with 0.3 g of AAtiram 75-S. Before germination the seeds were presoaked in water (4 hrs, 25°C). The germination was done within folded filter paper sheets.

Two seeds were put between each fold and the filter sheets, each containing 100 seeds were placed in a plastic tray (21x15x3 cm). After addition of 3 ml of water to each tray, the trays were covered with a plastic lid. Four trays were taken for each treatment and incubated at 20°C. After 7 and 14 days of incubation the degree of germination was estimated.

The results are summarized in Table 7.

The following examples 8 and 9 illustrate the inhibitory capacity of the ethenylimidazole compounds towards seedborn fungal infections.

## Example 8

Solutions B and D were compared with Panoctine Plus for the inhibition of seedborn Drechslera sorokiniana and D. teres.

40 g of barley seeds (a 1:3 mixture of Atem and Birgit) were mixed thoroughly with the fungicides in the way as described in the previous examples. The seeds were incubated with the so called TP-method (top of paper method). A piece of gray underfelt (quality T-10D, 730 g/$m^2$ from Schut en Zn, Heelsum) was placed in a perforated zinc tray of

11x27x2 cm. The underfelt was covered with white filter paper (type V 270, 132 $g/m^2$, Schut en Zn) and 50 seed kernels were put on top of the filter paper. The incubation was done in two ways:

a. 5 days at 10°C in the dark followed by 3 days at 20°C in the dark to evaluate the inhibition of D. sorokiniana,

b. 1 day at 20°C in the dark, then 1 day at -20°C in the dark in order to kill the seedlings and finally at 20°C for 7 days with exposure to near UV-light (12 hrs periods).

The relative humidity in both methods was 95-97%. In both incubation methods four trays were used for each test composition.

The results are given in Tables 8a and 8b.

Example 9

Solutions B and D were compared with AAtiram AT-S for the inhibition of Ascochyta pisi and Mycosphaerella pinodes in peas.

300 g of peas (Finale, bearing seedborn infection of Asc. pisi and Myc. pinodes) in a bottle of 750 ml were treated with the fungal formulations. The ethenylimidazoles were used in an amount of 2ml/kg and AAtiram AT-S in an amount of 3 g/kg. Untreated peas were used as a control. Mixing was affected in the usual way by means of a rotating wheel.

The BP. (between paper) method was used to incubate the seeds. Underfelt (type T-10D, 730 $g/m^2$, Schut en Zn) was placed into a perforated zinc tray (11x27x2 cm). The underfelt was covered with filter paper (type V 270, 132 $g/m^2$), then the peas (75 pieces per tray) were placed and covered with another V 270 filter paper. The seeds were incubated for 6 days in the dark at 20°C and a relative humidity of 95-97%.

The results are summarized in Table 9.

Example 10

In this example the effect of the ethenylimidazole compounds on the damping off of corn seeds was tested.

400 g of corn seeds (Dorina) were used for the treatments. Solutions A, B, C and D were added in an amount of 0.8 ml/kg and Captolate AC in an amount of 1.2 g/kg. The

mixing was effected in the same way as described previously, utilizing a rotating wheel (20 minutes, 25-30 rpm).

The seeds were sown into sandy soil (maximum moisture capacity 30% w/w) in plastic trays of 16x13x4 cm. The moisture content of the soil was brought to 22% (w/w) with tap water and then the tray was covered with a transparant plastic lid of 16x13x9 cm. For each treatment 4 trays were taken, each containing 75 seed kernels. The seeds were incubated for 14 days at 10°C, alternately in artificial daylight (fluorescent lamps) and in darkness (periods of 12 hrs). After 14 days the seedling emergence was evaluated.

The results are described in Table 10.

As can be seen the seeds have been heavily infected, but the treatment with the ethenylimidazole compositions improved the seedling emergence. Composition D was even far better than the standard Captolate AC.

Example 11

In this example the effect of varying amounts of 1-[2-(4-chlorophenyl)-3-methyl-1-pentenyl]-1H-imidazole hydrochloride (the Z-isomer) on the damping off of natively infected wheat was evaluated.

The following compositions were used:

D : containing 100 g/l of the ethenylimidazole compound and 10 g/l of diacetyl tartaric acid ester of mono- and diglycerides as a surface active agent.

D1 : the same as D, only containing 50 g/l of the ethenylimidazole compound instead of 100 g/l.

D2 : the same as D, only containing 10 g/l of the ethenylimidazole compound instead of 100 g/l.

D3 : the same as D, only containing 1 g/l of the ethenylimidazole compound instead of 100 g/l.

D4 : the same as D, only containing 200 g/l of the ethenylimidazole compound instead of 100 g/l.

Neo-Voronit (3 ml/kg) was used as a control composition.

To 40 g of wheat seeds (Marksman, with seed born fungal infection) in a 200 ml bottle 120 mcl (3 ml/kg) of test composition were added. After mixing thoroughly by means of a rotating wheel as described before, the seeds were sown into sandy soil in a plastic tray of 30x45x9 cm. The maximum moisture capacity of the soil was 38% (w/w). Tap water was added to the soil to a moisture content of 22% and then the tray was covered with a transparant plastic lid of 30x45x9 cm. Two trays, each containing 150 seed kernels, were incubated at 12°C in darkness and with exposure to artificial daylight (12 hrs periods) utilizing fluorescent lamps. After incubating for 17 days the degree of fungal attack was evaluated.

As standard treatments a control (without fungicide) and a treatment with Neo-Voronit (3 ml/kg) were utilized.

The results are summarized in Table 11.

A slight delay of the emergence of the seedling was observed, but no phytotoxicity. The inhibition of fungal attack was equal to or better than the standard treatment with Neo-Voronit.

## Example 12

In this example the control of fungal decay of natively infected wheat was studied. Solution C was compared with Neo-Voronit and a blanc.

The treatment and incubation procedures were the same as described in the previous example.

After 17 days of incubation at 12°C the degree of fungal attack was estimated. The results are given in Table 12. A slight delay of the emergence of the seedlings was observed, but the inhibition was equal to the standard treatment.

## Example 13

Commercially available plastic cheese coatings containing the fungicidal compounds were applied to freshly brined Gouda cheeses of 4 kg. The coating liquid was applied with a sponge in two stages. Firstly one side of the cheeses

was treated and after drying (the next day) the other side was treated. The cheeses were stored at 14°C and a relative humidity of 85-90%. During storage the cheeses were turned over every week.

Delvocid Instant, a 50% wetting powder formulation of natamycin and a control (plastic without a fungicide) were utilized as standards for comparison purposes. 6 Cheeses were used for each treatment.

After 11, 15 and 26 days of storage the appearance of the cheeses was evaluated. The results are summarized in Table 13. As can be seen 1-[2-(2,4-dichlorophenyl)-1-pentenyl)]-1H-imidazole hydrochloride is as effective as Delvocid for the conservation of cheeses.

## Example 14

This example shows the potency of the ethenylimidazole compounds to inhibit plant pathogen fungi. The minimal inhibitory concentration (i.e. the minimal concentration inhibiting growth of the micro-organism) of several ethenylimidazole compounds against various plant pathogens was determined by means of standard microbiological assays.

The results are shown in Table 14.

Compound A: 1-[2-(2,4-dichlorophenyl)-1-butenyl]-1H-imidazole hydrochloride.

Compound B: 1-[2-(2,4-dichlorophenyl)-1-pentenyl]-1H-imidazole hydrochloride

Compound C: 1-[2-(2,4-dichlorophenyl)-3-methyl-1-pentenyl]-1H-imidazole hydrochloride

Compound D: 1-[2-(4-chlorophenyl)-3-methyl-1-pentenyl]-1H-imidazole hydrochloride; Z-isomer;

Compound E: 1-[2-(4-chlorophenyl)-3-methyl-1-pentenyl]-1H-imidazole hydrochloride; E-isomer

Compound F: 1-[2-(4-chlorophenyl)-3-methyl-1-pentenyl]-1H-imidazole hydrochloride; E+Z isomers mixture.

Example 15

This example shows that the surface active agent used in the seed experiments have no fungicidal activity. By means of standard microbiological assays the minimal inhibitory concentrations of compound C and compound D with and without surface active agent were determined. The ratio of the surface active agent, diacetyl tartaric acid ester of mono- and diglycerides, to the fungicide is the same as in the seed experiments.

The results are summarized in Table 15.

Example 16

By means of standard microbiological assays the minimal inhibitory concentrations of compounds A, B and F against food spoiling yeasts and moulds were determined. The results are shown in Table 16.

Example 17

A powder composition was prepared by mixing the following ingredients

| | |
|---|---|
| 1-[2-(4-chlorophenyl)-3-methyl-1-pentenyl]-1H-imidazole hydrochloride (Z-isomer) | 10 g |
| sodium lauryl sulfate | 0.5 g |
| Aerosil 200 | 0.1 g |
| Dicalite | ad 100 g |

Example 18

A powder composition was prepared by mixing the following ingredients:

| | |
|---|---|
| 1-[2-(2,4-dichlorophenyl)-3-methyl-1-pentenyl]-1H-imidazole hydrochloride (Z + E isomer) | 5 g |
| Span 40 | 1 g |
| Aerosil | 0.1 g |
| Talc | ad 100 g |

Table 1

The influence of several fungicide compositions on the germination of corn seeds (Dorina).

| Fungicide composition | Amount added of the composition | Amount added of the active compound | % of germinated seeds after incubating for 7 days |
|---|---|---|---|
| A | 2 ml/kg | 200 ppm | 81 |
| B | 2 ml/kg | 200 ppm | 83 |
| C | 2 ml/kg | 200 ppm | 76 |
| D | 2 ml/kg | 200 ppm | 81 |
| Captolate AC | 3 g/kg | 1500/900 ppm | 82 |
| Blanc | - | - | 78 |

Table 2

The influence of several fungicide compositions on the germination of peas (Finale)

| Fungicide composition | Amount added of the composition | Amount added of the active compound | % of germinated seeds after incubating for 9 days |
|---|---|---|---|
| A | 2 ml/kg | 200 ppm | 88 |
| B | 2 ml/kg | 200 ppm | 96 |
| C | 2 ml/kg | 200 ppm | 86 |
| D | 2 ml/kg | 200 ppm | 93 |
| AAtiram AT-S | 3 g/kg | 1500/900 ppm | 97 |
| Blanc | - | - | 97 |

## Table 3

The influence of several fungicide compositions on the germination of barley (Banteng)

| Fungicide composition | Amount added of the formulation | Amount added of the active compound(s) | % of germinated seeds after incubating for 7 days (20°C) |
|---|---|---|---|
| A | 3 ml/kg | 300 ppm | 92 |
| B | 3 ml/kg | 300 ppm | 92 |
| C | 3 ml/kg | 300 ppm | 92 |
| D | 3 ml/kg | 300 ppm | 96 |
| Panoctine Plus | 2 ml/kg | 600/50 ppm | 95 |
| Blanc | - | - | 99 |

## Table 4

The influence of several fungicide compositions on the germination of wheat (Caribo)

| Fungicide composition | Amount added of the composition | Amount added of the active compound(s) | % of germinated seeds after incubating for 7 days at 20°C |
|---|---|---|---|
| A | 3 ml/kg | 300 ppm | 97 |
| B | 3 ml/kg | 300 ppm | 96 |
| C | 3 ml/kg | 300 ppm | 97 |
| D | 3 ml/kg | 300 ppm | 98 |
| Neo-Voronit | 3 ml/kg | 1000/20 ppm | 97 |
| Blanc | - | - | 97 |

Table 5

The influence of several fungicide compositions on the germination of sugar beet seeds.

| Fungicide composition | Amount added of the formulation | Amount added of the active compound | % of germinated seeds after incubating for 7 days | % of germinated seeds after incubating for 14 days |
|---|---|---|---|---|
| A | 8 ml/kg | 800 ppm | 81 | 83 |
| B | 8 ml/kg | 800 ppm | 88 | 91 |
| C | 8 ml/kg | 800 ppm | 83 | 86 |
| D | 8 ml/kg | 800 ppm | 84 | 85 |
| AAtiram 75 S | 6 g/kg | 4500 ppm | 83 | 87 |
| Blanc | - | - | 80 | 82 |

Table 6

The influence of several fungicide compositions on the germination of garden cress seeds (Gromono)

| Fungicide composition | Amount added of the formulation | Amount added of the active compounds | % of germinated seeds after incubating for 2 days | % of germinated seeds after incubating for 10 days |
|---|---|---|---|---|
| A | 2 ml/kg | 200 ppm | 85 | 95 |
| B | 2 ml/kg | 200 ppm | 87 | 92 |
| C | 2 ml/kg | 200 ppm | 92 | 97 |
| D | 2 ml/kg | 200 ppm | 92 | 95 |
| Blanc | - | - | 95 | 97 |

Table 7

The influence of several fungicide compositions on the germination of mangel seeds (Capax).

| Fungicide composition | Amount added of the composition | Amount added of the fungicide | % of germinated seeds after incubating for 7 days | % of germinated seeds after incubating for 14 days |
|---|---|---|---|---|
| B | 16 ml/kg | 1600 ppm | 83 | 85 |
| B1 | 16 ml/kg | 800 ppm | 80 | 81 |
| B2 | 16 ml/kg | 400 ppm | 86 | 87 |
| B3 | 16 ml/kg | 80 ppm | 84 | 86 |
| B4 | 16 ml/kg | 8 ppm | 83 | 85 |
| AAtiram 75 S | 6 g/kg | 4500 ppm | 87 | 89 |
| Blanc | - | - | 86 | 87 |

Table 8a

The inhibitory effect of several fungicides on seedborn Drechslera sorokiniana in barley. Incubation conditions: 5 days at 10°C and 3 days at 20°C.

| Formulation | Amount of formulation (c.q. fungicide) added | | % of seeds contaminated by D.sorokiniana |
|---|---|---|---|
| B | 3 ml/kg | (300 ppm) | 2.5 |
| D | 3 ml/kg | (300 ppm) | 1.0 |
| Panoctine Plus | 2 ml/kg | (600/50 ppm) | 1.5 |
| Blanc | - | ( - ) | 15.5 |

Table 8b

The inhibitory effect of several fungicides on seedborn D.sorokiniana and D. teres in barley. Incubation successively 1 day at 20°C, 1 day at -20°C and 7 days at 20°C.

| Formulation | Amount of formulation (c.q. fungicide) added | | % of seeds contaminated by D. teres | by D.sorokiniana |
|---|---|---|---|---|
| B | 3 ml/kg | (300 ppm) | 1.0 | 0.5 |
| D | 3 ml/kg | (300 ppm) | 4.0 | 1.0 |
| Panoctine Plus | 2 ml/kg | (600/50 ppm) | 4.5 | 2.0 |
| Blanc | - | ( - ) | 60.5 | 26.5 |

Table 9

The inhibition of seedborn Ascochyta pisi and Mycosphaerella pinodes in peas by several fungicides. Incubation at 20°C for 6 days.

| Formulation | Amount of formulation (c.q. fungicide) added | | % of seeds contaminated by A.pisi/ M.pinodes | by Saprophytes |
|---|---|---|---|---|
| B | 2 ml/kg | (200 ppm) | 4 | 2 |
| D | 2 ml/kg | (200 ppm) | 4 | 2 |
| AAtiram AT-S | 3 g/kg | (1500/900 ppm) | 3 | 4 |
| Blanc | - | ( - ) | 13 | 6 |

0092278

Table 10

The degree of seedling emergence of natively infected corn seeds after incubation for 14 days at 10°C.

| Fungicide composition | Amount of composition (c.q. fungicide) added | % of seedling emergence after 14 days |
|---|---|---|
| A | 2 ml/kg (200 ppm) | 20 |
| B | 2 ml/kg (200 ppm) | 20 |
| C | 2 ml/kg (200 ppm) | 14 |
| D | 2 ml/kg (200 ppm) | 42 |
| Captolate AC | 3 g/kg (1500/900 ppm) | 25 |
| Blanc | - ( - ) | 11 |

Table 11

The influence of different concentrations of 1-[2-(4-chlorophenyl)-3-methyl-1-pentenyl]-1H-imidazole (Z-isomer) on fungal decay of wheat.

| Formulation | Amount of formulation (c.q. fungicide) added | % of seeds contaminated by fusarium species |
|---|---|---|
| D | 3 ml/kg (300 ppm) | 35 |
| [illegible] | 3 ml/kg (150 ppm) | 35 |
| [illegible] | 3 ml/kg ( 30 ppm) | 50 |
| [illegible] | 3 ml/kg ( 3 ppm) | 47 |
| [illegible]4 | 3 ml/kg (600 ppm) | 26 |
| Neo- ronit | 3 ml/kg (1000/20 ppm) | 31 |
| Blanc | - ( - ) | 64 |

Table 12

The influence of 1-[2-(2,4-dichlorophenyl)-3-methyl-1-pentenyl] -1H-imidazole hydrochloride on fungal decay of wheat.

| Formulation | Amount of formulation (c.q. fungicide) added | % of seeds contaminated by fungi |
|---|---|---|
| C | 3 ml/kg (300 ppm) | 35 |
| Neo-Voronit | 3 ml/kg (1000/20 ppm) | 32 |
| Blanc | - ( - ) | 65 |

Table 13

Percentage surface area of Gouda cheeses covered with mold at different stages of curing time (14°C and 85% relative humidity).

| Cheese coated with | % Area covered with mold after | | |
|---|---|---|---|
| | 11 days | 15 days | 26 days *) |
| Plastic cheese coating only | 0 | 0-5 | 20-30 (24) |
| Cheese coating containing Delvocid Instant (1000 ppm) | 0 | 0 | 0-10 (6) |
| Cheese coating containing 1- [2-(2,4-dichlorophenyl)-1-pentenyl]-1H-imidazole hydrochloride (500 ppm) | 0 | 0-1 | 0-10 (7) |

*) mean values in parentheses.

Table 14

The minimal inhibitory concentration of ethenylimidazole compounds against plant pathogen fungi (mcg/ml)

| Plant pathogen | Compound A | B | C | D | E | F |
|---|---|---|---|---|---|---|
| Alternaria alternata CBS 103.33 | 3 | 0.4 | 0.4 | 0.75 | 0.75 | 0.4 |
| Aspergillus flavus CBS 625.66 | 12.5 | 3 | 12.5 | 12.5 | 12.5 | 12.5 |
| Botrytis cinera | 0.75 | 0.4 | 0.4 | 1.5 | 0.4 | - |
| Ceratocystis paradoxa CBS 453.66 | ≤0.05 | ≤0.05 | ≤0.05 | ≤0.05 | ≤0.05 | 3 |
| Cercospora musae CBS 143.36 | ≤0.05 | ≤0.05 | ≤0.05 | ≤0.05 | ≤0.05 | - |
| Chondrostereum purpureum CBS 367.58 | 3 | 0.75 | ≤0.05 | 0.75 | 0.75 | - |
| Fusarium (isolated from tulip bulbs) A3241 | 3 | 0.4 | 12.5 | 12.5 | 0.75 | 0.75 |
| Fusarium oxysporum f. sp. lycopersici A3247 | 3 | 0.75 | 25 | 100 | 0.75 | 0.75 |
| Fusarium solani A3245 | 1.5 | 0.4 | 12.5 | 50 | 0.75 | 0.75 |
| Helminthosporium solani CBS 365.75 | 0.2 | ≤0.05 | ≤0.05 | 0.2 | ≤0.05 | - |
| Penicillium italicum CBS 278.58 | ≤0.05 | ≤0.05 | ≤0.05 | 0.2 | 0.1 | ≤0.05 |
| Phoma exigua CBS 431.74 | 0.75 | ≤0.05 | ≤0.05 | 0.4 | 0.75 | 0.2 |
| Phytophthora cactorum CBS 435.64 | 1.5 | 0.2 | ≤0.05 | 0.4 | 0.4 | 0.75 |
| Septoria avenae CBS 516.74 | 0.2 | ≤0.05 | ≤0.05 | 0.4 | 0.1 | - |
| Venturia inaequalis CBS 815.69 | ≤0.05 | ≤0.05 | ≤0.05 | ≤0.05 | ≤0.05 | ≤0.05 |
| Vert illium albo-atrum CE 382.66 | ≤0.05 | ≤0.05 | ≤0.05 | ≤0.05 | ≤0.05 | - |
| Phyti acheiphilum CB - 0.77 | - | - | - | - | - | 0.75 |

Table 15

The minimal inhibitory concentration of 1-[2-(2,4-dichlorophenyl)-3-methyl-1-pentenyl]-1H-imidazole hydrochloride and 1-[2-(4-chlorophenyl)-3-methyl-1-pentenyl]-1H-imidazole hydrochloride (Z-isomer) without and with a surface active agent against some plant pathogenic fungis.

| Plant pathogenic fungi | Minimal inhibitory concentration (mcg/ml) | | | | |
|---|---|---|---|---|---|
| | C | C + surface active agent | D | D + surface active agent | surface active agent |
| Aspergillus flavus | 3 | 3 | 25 | 25 | >200 |
| Fusarium solani | 0.4 | 0.4 | 25 | 25 | >200 |
| Penicillium italicum | ≤0.05 | ≤0.05 | 0.1 | 0.1 | >200 |
| Phytophthora cactorum | 0.4 | 0.75 | 1.5 | 1.5 | >200 |

Table 16

Minimal inhibitory concentrations against food spoiling yeasts and moulds.

| MOULDS | Minimal inhibitory concentration (mcg/ml) of | | |
|---|---|---|---|
| | A | B | F |
| Alternaria alternata | 0.2 | 0.1 | 0.2 |
| Aspergillus species | 0.004-1.5 | 0.004-2 | 0.01-12.5 |
| Botrytis cinera | 0.75 | 0.05 | 0.1 |
| Byssochlamys nivea | 3 | 0.75 | 12.5 |
| Fusarium sp. | 0.1-3 | 0.004-0.2 | 0.05-0.75 |
| Geotrichum candidum | 50 | 25 | 6 |
| Mucor hiemalis | 6 | 6 | 12.5 |
| Penicillium sp. | 0.004-0.4 | 0.004-0.1 | 0.01-0.75 |
| Rhizopusarrhizus | 0.4 | 0.004 | 0.75 |
| Scopulariopsis fusca | 1.5 | 0.2 | 3 |
| Syncephalastrum racemosum | 6 | 1.5 | 1.5 |
| Trichodermo viride | 6 | 0.4 | 12.5 |
| YEASTS | | | |
| Debaryomyces hansenii | 25 | 12.5 | 0.75 |
| Hyphopichia burtonii | 12.5 | 3 | 0.2 |
| Hansenula anomala | 12.5 | 12.5 | 0.75 |
| Hansenula sulpelliculosa | 6 | 1.5 | ⩽0.05 |
| Pichia membranafaciens | 50 | 25 | 3 |
| Saccheromyces cerevisiae | 6 | 0.2 | - |
| Saccharomyces bailii | 50 | 25 | 0.75 |
| Saccharomyces ludwigii | 3 | ⩽0.1 | ⩽0.05 |
| Brettanomyces anomalus | 1.5 | ⩽0.1 | 0.1 |
| Schizosaccharomyces pombe | 6 | 0.4 | 0.4 |
| Candida sp. | 25-50 | 12.5-25 | 0.2-3 |
| Kluyveromyces lactis | 12.5 | 1.5 | 0.1 |
| Zygosaccharomyces rouxii | 12.5 | 6 | 0.2 |

0092278

Claims

1. Process for combatting fungal infections in agricultural products and foodstuffs, characterized in that they are treated with a compound of the formula

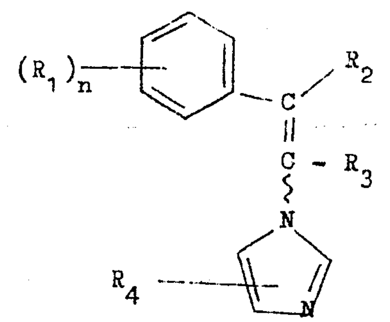

I

in which n is 0, 1 or 2, $R_1$ represents a halogen atom, a trifluoromethyl group or an alkyl or alkoxy group with at most 6 carbon atoms, the two substituents being the same or different when n is 2, $R_2$ represents an alkyl group with 1 to 6 carbon atoms, an alkenyl group with 2 to 4 carbon atoms or a cycloalkyl group with 3 to 6 carbon atoms, $R_3$ represents a hydrogen atom, an alkyl group with 1 to 6 carbon atoms or an alkenyl group with 2 to 4 carbon atoms and $R_4$ represents a hydrogen atom, an alkyl group with 1 to 6 carbon atoms or a nitro group, or an acid addition salt thereof.

2. Process according to claim 1 in which a compound of formula I or an acid addition salt thereof is used in which $(R_1)_n$ is 4-chloro or 2,4-dichloro, $R_2$ is ethyl, propyl or sec-butyl and $R_3$ and $R_4$ are hydrogen atoms.

3. Process according to claim 2 in which 1-[2-(2,4-dichlorophenyl)-1-pentenyl]-1H-imidazole or an acid addition salt thereof is used.

4. Process according to claim 1, 2 or 3, for the treatment of seeds, characterized in that 1-10,000 ppm by weight of active substance is used.

5. Process according to claim 4,characterized in that 10-2000 ppm of active substance is used.

6. Process according to claim 1, 2 or 3 for the treatment of tubers and bulbs, characterized in that 0.2-1500 ppm by weight of active substance is used.

7. Process according to claim 6, characterized in that 2-300 ppm of active substance is used.

8. Process according to claim 1, 2 or 3 for the treatment of cheese, characterized in that 0.5-500 mcg of active substance per $cm^2$ of product is used.

9. Process according to claim 8, characterized in that 2-20 mcg/$cm^2$ of active substance is used.

10. Compositions for combatting fungal infections in agricultural products and food stuffs, which comprise as the active ingredient a compound of formula I, as defined in claim 1, or an acid addition salt thereof.

11. Compositions as claimed in claim 10, which comprise as the active ingredient a compound of formula I in which $(R_1)_n$ is 4-chloro or 2,4-dichloro, $R_2$ is ethyl, propyl or sec-butyl and $R_3$ and $R_4$ are hydrogen atoms, or an acid addition salt thereof.

12. Compositions as claimed in claim 11, which comprise as the active ingredient 1-[2-(2,4-dichlorophenyl)-1-pentenyl]-1H-imidazole or an acid addition salt salt thereof.

13. Agricultural products and foodstuffs treated according to any of claims 1-9.

0092278

European Patent Office

# EUROPEAN SEARCH REPORT

Application number

EP 83 20 0528

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP-A-0 003 732 (THE WELLCOME FOUNDATION) * Pages 2-4 * | 1-3 | A 01 N 43/50 // C 07 D 233/94 C 07 D 233/93 C 07 D 233/92 C 07 D 233/58 |
| A | FR-A-1 486 817 (BASF) * Page 1 * | 1 | |
| E | EP-A-0 060 223 (CIBA-GEIGY) * Pages 1-10, 25-31, 53, no. 2.7 * | 1-3 | |
| D,E | EP-A-0 049 913 (GIST-BROCADES) * Pages 24-25, example 16; pages 52-53 * | 1-3 | |
| | | | TECHNICAL FIELDS SEARCHED (Int. Cl. 3) |
| | | | C 07 D 233/00 A 01 N 43/00 |

The present search report has been drawn up for all claims

| Place of search | Date of completion of the search | Examiner |
|---|---|---|
| THE HAGUE | 30-06-1983 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82